# EUROPÄISCHE PATENTANMELDUNG

(11) **EP 2 998 386 A2**
(43) Veröffentlichungstag der Anmeldung: **23.03.2016**
(21) Anmeldenummer: 15002693.8
(22) Anmeldetag: 17.09.2015
(51) Int. Cl.: C12M 1/107, C12M 1/00, C12P 5/02

(54) **VERFAHREN ZUM BETRIEB EINER BIOGASANLAGE**

(30) Priorität: 17.09.2014 DE 102014013777
(71) Anmelder: Rogmans, Maria, 47546 Kalkar (DE)
(72) Erfinder: Wilhelm, Hermann-Josef, 47546 Kalkar (DE)
(74) Vertreter: Schmidt, Karl Michael

(57) **Zusammenfassung**

Die Erfindung betrifft ein Verfahren zum Betrieb einer Biogasanlage bei welcher landwirtschaftliche Gülle und/oder in einem vorherigen Gärprozess bereits teilausgegaster Gärbrei, und/oder Klärschlamm, und/oder Abfälle aus der Lebensmittelproduktion als Gärssubstrat in mindestens einem Gärprozess vergärt oder nachvergärt wird, wobei der landwirtschaftlichen Gülle und/oder dem Gärbrei oder dem teilausgegasten Gärbrei, und/oder dem Klärschlamm, und/oder den Abfällen aus der Lebensmittelproduktion ein die Biogasproduktion unterstützendes Stoffgemisch, d.h. Biogasbooster beigefügt wird, welches zuvor hergestellt wird, und das/der Stoffgemisch/Biogasbooster aquatischen Pflanzen mit Beimengungen von Fettsäuren und/oder Fettsäureestern enthält, sowie ein Verfahren zur Herstellung eines solchen Boosters. Um hierbei eine verbesserte Adaption dieses Systems an verschiedene Biogasanlagen zu erreichen ist erfindungsgemäß vorgeschlagen, dass zusätzlich Eisenhydroxid, und/oder PH-Regulator, und/oder Zeolite, und/oder verseifte Rückstande aus der Lebensmittel- oder Speiseölproduktion, und/oder Enzyme, und/oder Mineralien, und/oder gemahlene Aktiv- und/oder Holzkohle dem besagten Stoffgemisch hinzugefügt wird.

## Beschreibung

Die Erfindung betrifft ein Verfahren zum Betrieb einer Biogasanlage bei welcher landwirtschaftliche Gülle und/oder in einem vorherigen Gärprozess bereits teilausgegaster Gärbrei, und/oder Klärschlamm, und/oder Abfälle aus der Lebensmittelproduktion als Gärssubstrat in mindestens einem Gärprozess vergärt oder nachvergärt wird, wobei der landwirtschaftlichen Gülle und/oder dem Gärbrei oder dem teilausgegasten Gärbrei, und/oder dem Klärschlamm, und/oder den Abfällen aus der Lebensmittelproduktion ein die Biogasproduktion unterstützendes Stoffgemisch, d.h. Biogasbooster beigefügt wird, welches zuvor hergestellt wird, und das/der Stoffgemisch/Biogasbooster aquatische Pflanzen mit Beimengungen von Fettsäuren und/oder Fettsäureestern enthält, sowie ein Verfahren zur Herstellung eines solchen Boosters, gemäß Oberbegriff der Patentansprüche 1, 5, 7 und ein solches Stoffgemisch/Biogasbooster gemäß Oberbegriff des Patentanspruches 8.

Biogasanlagen mit aquatischen Pflanzen als Gärsubstrat zu betreiben ist als solches aus der DE 2008 050 974 A1 vom selben Anmelder bekannt.
Dabei werden die aquatischen Pflanzen in gestapelten Wannensystemen kultiviert und als Gärsubstrat zur Biogaserzeugung genutzt.

Dabei hat sich aber auch gezeigt, dass der ausschließliche Betrieb einer solchen aquatischen Kultureinrichtung nur zur Biogaserzeugung wegen des benötigten Wärmehaushaltes und der baulichen Maßnahmen in wirtschaftlicher Hinsicht kritisch sein könnte.
Somit macht es Sinn, dieselbe Kultureinrichtung zeitgleich mit der Biomassenpropagation als solcher noch einem weiteren Zweck, nämlich dem der Abwasserbehandlung zuzuführen.

In diesem Zyklus aber spielen eingesetzte Güllen im Stoffstrom eine weitere Rolle. Außerdem löst die Benutzung aquatischer Pflanzen den Einsatz anderer Biogassubstrate nicht gänzlich ab. Vielmehr wird erreicht, dass der Primäreinsatz von Biogassubstraten mit Hilfe dieses Boosters" reduziert werden kann.
Dies ist aus der DE 10 2012 024 108 A1 bekannt.

Die Verwertung von Gülle in Biogasanlagen ist ebenfalls bekannt. Dabei ist es auch bekannt Gülle entweder zusätzlich, bspw als Impfgülle einzusetzen, oder die Biogasanlage teilweise auf Gülle als Gärsubstrat zu betreiben.
Mit Hilfe des Biogas-Boosters aus dem oben genannten Stand der Technik sind die oben genannten hohen zusätzlichen Biogasausbeuten erzielbar. Außerdem erfolgt der Biogasaufschluss auch so schnell, dass dies der extrem schnellen Verfügbarkeit der besagten aquatischen Pflanzen Rechnung trägt.

Das besagte Verfahren kann vorteilhaft über einen ganz erheblichen Mischungsbereich gefahren werden. Dieser ist so ausgeführt, dass der Anteil der Mischung aus aquatischen Pflanzen plus Biogasbooster im Gärbrei der Biogasanlage in Summe bei ca 1% bis 90% der Feuchtmasse des Gärbreies der im Gärraum eingebrachten Dickphase liegt. Die Wahl dieses weiten Bereiches basiert darauf, dass die oben genannten funktionalen Konsequenzen auf erfindungsgemäße Weise miteinander in Wirkung gebracht werden können.
Der hohe und extrem schnelle Massenzuwachs und die Stoffzusammensetzung des Biogas-Boosters ermöglichen das ertragreiche Fahren dieses weiten Mischungsbereiches erst. Das Besondere liegt hierbei also in dem möglichen weiten Mischungsbereich, in welchem die genannten Vorteile erzielbar sind. Vorteilhaft ist aber oftmals ein Mengenanteil von 1,5% Booster im Gärbrei.
Die Menge im Booster eingesetzter aquatischer Pflanzen kann dabei auf von 5% bis 60 % des Boosters beschränkt werden.
Der Mehrertrag durch den dadurch geboosterten restlichen Biogasauschluss ist so groß, dass sich eine Kulturanlage mit aquatischen Pflanzen wieder wirtschaftlich sehr gut darstellt, wenn wie oben ausgeführt, die Kulturanlage der aquatischen Pflanzen gleichzeitig zur Reinigung von Abwässern aus Biogasanlagen herangezogen wird.

Die Anwendung des beschriebenen bekannten Verfahrens ist an sich schon vorteilhaft. Da aber jede Biogasanlage individuell ist, und teilweise sogar über eine individuelle Mikrobiologie, d.h. Bakterienstämme verfügt, ist die Adaption des bekannten Systems an verschiedene Biogasanlagen nicht trivial. Bisher sind keine Maßnahmen hierzu bekannt. Wichtig ist aber eine vollständige Adaption des Systems an verschiedene Biogasanlagen und deren Substratsysteme und Stoffströme.

Der Erfindung lag daher die Aufgabe zugrunde, ein Verfahren sowie ein die Biogasproduktion unterstützendes Stoffgemisch, d.h. Biogasbooster derart weiter zu entwickeln, dass eine vollständige Adaption an verschiedene Biogasanlagen möglich ist, mit einer Erhöhung des Wirkungsgrades in Bezug auf den Aufschluss von Biogas aus verwendetem Biogassubstrat möglich ist.

Die gestellte Aufgabe wird bei einem Verfahren der gattungsgemäßen Art erfindungsgemäß durch die kennzeichnenden Merkmale des Anspruches 1 gelöst.

Vorteilhafte Ausgestaltungen der erfindungsgemäßen Anwendung sind in den abhängigen Ansprüch 2 bis 4 angegeben.

Ein Verfahren zur Herstellung des Biogassubstrates ist in Anspruch 5 angegeben.

Ein Verfahren zur Adaption des Biogasboosters an eine individuelle Biogasanlage ist in Anspruch 7 angegeben.

Ein Stoffgemisch oder Booster der verwendeten Art ist in Anspruch 8 angegeben.

Die übrigen abhängigen Ansprüche enthalten sinnreiche Ausgestaltungen.

Kern der verfahrensgemäßen Erfindung in einer ersten Ausgestaltung ist, dass zusätzlich Eisenhydroxid, und/oder PH-Regulator, und/oder Zeolite, und/oder verseifte Rückstande aus der Lebensmittel- oder Speiseölproduktion, und/oder Enzyme, und/oder Mineralien, und/oder gemahlene Aktiv- und/oder Holzkohle dem besagten Stoffgemisch hinzugefügt wird.

Bei der vorliegenden Zusammensetzung des den Biogasprozesse unterstützenden oder verbessernden Stoffgemisches kommt es darauf an, dass nunmehr noch zusätzlich Eisenhydroxid, sowie weitere Regulatoren mit hinzugegeben werden. Auch die Zeolithe haben in Kombination mit dem Einsatz des teilweise aus aquatischen Pflanzen bestehenden Stoffgemisches zur Unterstützung der Biogasproduktion ein vorteilhaftes Zusamenwirkungspiel. Im Gegensatz zu einem einfachen Gemisch biogastypischer Hilfsstoffe ist die Mischung gemäß der vorliegenden Erfindung reaktionsmäßig nochmals durch die Komponente "aquatische Pflanzen" so gepuffert, dass die übrigen Substanzen homogen in den Gärbrei zuführbar sind.

In weiterer vorteilhafter Ausgestaltung ist angegeben, dass dieses, die Biogasproduktion unterstützende Stoffgemisch, einem Nachfermentationsprozess einer Biogasanlage zugeführt wird. Auf diese Weise werden die aus dem Hauptfermenter entnommenen Biomassen wieder aufbereitet in den Nachfermentationsprozess gegeben, um eine optimale Nachvergärung und einen möglichst vollständigen Aufschluss der noch nicht vergasten Bestandteile des Gärbreies zu erreichen.

In alternativer oder aber auch kumulativer Ausgestaltung ist angegeben, dass dieses, die Biogasproduktion unterstützende Stoffgemisch, einem Hauptfermentationsprozess einer Biogasanlage zugeführt wird.

Weiterhin ist für all diese Verfahrensvarianten vorgesehen, dass das die Biogasproduktion unterstützende Stoffgemisch dem Haupt- oder Nachfermenter der Biogasanlage in einem Zeittakt von 6 bis 30 Stunden in wiederholten Dosen zugeführt wird, derart dass der Methangastrieb aus dem Gärbrei dauerhaft angehoben und gehalten wird, und eine Biomassezufütterung zeitlich parallel kontinuierlich so nachgeführt wird, dass der gleiche Methangastrieb bei mengenmäßig reduzierter Biomassenzufütterung erhalten bleibt.

In weiterer vorteilhafter Ausgestaltung ist vorgesehen, dass bei der Produktion der aquatischen Pflanzen die beim Betrieb der Biogasanlage und/oder die beim Betrieb eines BHKWs anfallende Abwärme zur Beheizung einer Kulturanlage zur Produktion der aquatischen Pflanzen eingesetzt wird, und dass das in der Biogasproduktion oder beim BHKW-Betrieb anfallende CO2 der Kulturanlage als vorzugeweise gasförmiger Düngestoff zugeführt.

Wichtig dabei ist, dass die verwendete Kultureinrichtung eine tägliche Erntemenge aquatischer Pflanzen produziert, so dass in den angegebenen Zeittakten auch die notwendigen Mengen für die tägliche Boosterung zur Verfügung steht. In dieser Kulturanlage wird die Abwärme aus dem Biogasprozess und/oder einem BHKW eingesetzt um die aquatischen Pflanzen zu produzieren. Ferner wird auch anfallendes CO2 als gasförmiger Düngestoff in der Kulturanlage eingesetzt.

Von besonderer Bedeutung im Zusammenhang mit der Erfindung ist ein Verfahren zur Herstellung des Biogasboosters oder des den Biogasprozess unterstützenden Stoffgemisches.
Es ist ein Verfahren zur Herstellung des die Biogasproduktion in einer Biogasanlage unterstützenden Stoffgemisches oder Boosters, welches im wesentlichen aus einem Gemisch besteht, dessen erste Komponente aus einer oder mehreren aquatischen Pflanzen besteht, und dessen zweite Komponente Fette, und/oder Fettsäuren besteht. Dabei werden die aquatischen Pflanzen zunächst als Frischmasse eingesetzt, und durch Zugabe eines trockenen oder teilgetrockneten hydrophilen Pflanzenmehles teilentwässert, und zu einem Gemisch mit entsprechend resultierendem Trockensubstanzgehalt vermengt, dessen resultierender Trockensubstanzgehalt höher ist als der Trockensubstanzgehalt der eingesetzten aquatischen Pflanzen. Mit diesem Schritt wird folgende technische Maßgabe auf sehr einfache Weise erfüllt. Wie bereits aus der DE 10 2012 024 108 A1 bekannt, wird die aus aquatischen Pflanzen bestehende Pflanzenfrischmasse zunächst zumindest teilgetrocknet, um den Pflanzenkörper wieder aufnahmefähig für Flüssigkeiten, auch flüssige Fette wie Glycerine zu machen. Diese hinzu gegebenen flüssigen Fette, oder auch andere Flüssigkeiten sollen nämlich bei der Vermengung mit der aquatischen Pflanzenmasse, von derselben aufgenommen, quasi aufgesaugt werden. Diese Aufnahme von bspw Glycerinen in den angetrockneten Pflanzenkörper der aquatischen Biomasse hat sich als in erheblichem Maße effektiv bei der anschließenden Steigerung der Biogasproduktionsrate des noch nicht vollständig ausgegasten Gärbreies gezeigt.

Dem so vorbereiteten Gemisch werden sodann in weiterer vorteilhafter Ausgestaltung folgende weitere Stoffe mit zugegeben: Eisenhydroxid, und/oder PH-Regulator, und/oder Zeolite, und/oder verseifte Rückstande aus der Lebensmittel- oder Speiseölproduktion, und/oder Enzyme, und/oder Mineralien, und/oder gemahlene Aktiv- und/oder Holzkohle.

Das die Biogasproduktion steigernde Stoffgemisch mit aquatischen Pflanzen, oder auch Booster genannt, erzeugt einen schnellen Gasaufschluss.

Wichtig ist bei der Anwendung dieser Verfahren noch das nachfolgend beschriebene Verfahren, bei welchem dem Stoffgemisch, auch Booster genannt, noch auf die jeweilige zu boosternde Biogasanlage speziell adaptierte und überpopulierte Bakterienstämme in einen separaten Prozess quasi erbrütet werden, und dem Stoffgemisch, d.h. dem Booster mit beigemischt werden, bevor dieses Stoffgemisch oder dieser Booster der zu boosternden Biogasanlage mit zugeführt wird.
Bedeutsam kann auch der Einsatz von Archaea, d.h. Archaebakterien sein.

Von erheblicher Wichtigkeit sind daher folgende erfindungsgemäßen Verfahrensmaßnahmen zum Betrieb einer Biogasanlage. Hierbei wird landwirtschaftliche Gülle und/oder in einem vorherigen Gärprozess bereits teilausgegaster Gärbrei, und/oder Klärschlamm, und/oder Abfälle aus der Lebensmittelproduktion als Gärssubstrat in mindestens einem Gärprozess vergärt oder nachvergärt, wobei der landwirtschaftlichen Gülle und/oder dem Gärbrei oder dem teilausgegasten Gärbrei, und/oder dem Klärschlamm, und/oder den Abfällen aus der Lebensmittelproduktion ein die Biogasproduktion unterstützendes Stoffgemisch beigefügt wird, welches zuvor hergestellt wird.

Der Biogasanlage wird zunächst ausgegaster oder teilausgegaster Gärbrei entnommen, und in ein gesondertes bakteriologisches Brutgefäß, hier auch Testfermenter genannt, eingebracht und dem besagten Stoffgemisch, d.h. Booster hinzugeführt, indem in einem Batchverfahren zunächst der Gärbrei zusammen mit dem besagten Stoffgemisch über einen Zeitraum von 1 bis 45 Tagen bei einer Temperatur von etwa 30°C bis etwa 42 °C oder sogar höher, anaerob kultiviert wird, derart, dass sich diejenigen Bakterienstämme aus dem Gemisch vorhandender Bakterienstämme überproportional entwickeln, die genau an das Gemisch des entnommenen Gärbreies mit dem Stoffgemisch adaptiert haben, und dass diesem so bakteriell erbrüteten Gärbrei Teilmengen entnommen werden und als bakteriologische Impfmasse in das finale Stoffgemisch oder direkt in den Haupt- oder Nachfermentationsprozess mit eingemischt werden.

Mit anderen Worten heisst dies, dass der ansonsten weiterlaufenden Biogasanlage ausgegaster oder teilausgaster Gärbrei in einer Teilmenge entnommen wird, und einem separaten Brutgefäß zugeführt wird, in welchem der oben bereits genannte Booster mit zugegeben wird.
In diesem Brutgefäß bewirken nun die Boosterbestandteile eine überproportionale Entwicklung solcher Bakterienstämme im Gärbrei, die genau an die noch nicht aufgeschlossenen Bestandteile im Gärbrei adaptiert werden. Der so laufende parallele Batchprozess dauert ca 4 bis 6 Wochen an, bis sowohl der Booster selbst, als auch der Gärbrei darin möglichst gänzlich ausgegast sind. Die unter diesen Bedingungen populierten Bakterienstämme sind an die nur schwer aufschließbaren Gärbreibestandteile adaptiert und zahlenmäßig überpopuliert. Diese werden in Teilmengen aus dem Brutgefäß entnommen und dem o.g. Stoffgemisch, d.h. Booster, zugemischt, und dann in oben genanntem Mengenverhältnis bspw dem Nachfermentionsprozess, d.h. den täglichen Transfermengen zwischen Hauptfermenter und Nachfermenter zugemischt.
Die Entnahme aus dem Brutgefäß und die Mischung in das Stoffgemisch/Booster erfolgt unter weitestgehend anaeroben Bedingungen, oder nur mit sehr kurzer oberflächlicher Belüftung.

Wichtig ist dabei folgende Erkenntnis. Mit dem so konditionierten Stoffgemisch, d.h. Booster, ist es möglich den Biogasproduktionsprozess an sich, aber vor allem den Nachvergärungsprozess mit einer höheren Ausgasungsrate zu betreiben. Es hat sich gezeigt, dass die so vorgenommene Boosterung einen vollständigeren Gasaufschluss von Gärresten, die aus dem Hauptfermentationsprozess kommen, in dem nachfolgenden Nachfermentationsprozess erzielt.
Dies wird bewirkt, weil die Textur der verwendeten aquatischen Pflanzen, zusammen mit den genannten Stoffen beispielsweise mit in den Gärbreitransfer zwischen Hauptfermenter und Nachfermenter injiziert werden kann. Das biologisch nachbelebte Stoffgemisch wirkt extrem homogen auf den Gärbrei bspw im Nachfermenter ein, weil Hilfsstoffe und einsteilige Energieträger in einer biogenen Hülle, die von den aquatischen Pflanzen gebildet wird, in fein verteilten Mikrodosen in den Gärbrei eingeschleust werden. D.h. die quasi biogene Verpackung der Hilfs- und Wirkstoffe in eine selbst auch schnell aufzehrende biologische Pflanzenhülle bewirkt hier einen geboosterten, insbesondere Nachfermentations-Prozess. Das Ergebnis ist ein signifikant höherer Gasertrag, insbesondere bei der Nachfermentation.
Die Pflanzenhülle wird gebildet durch bspw Wasserlinsen (Lemnaceae) oder Wasserhyazinthe (Eichhornia) aber auch anderen aquatischen Pflanzen. Grundsätzlich möglich sind natürlich auch Algen. Insbesondere Wasserlinsen selbst haben einen so schnellen Gasaufschluss bei der Biogasproduktion, dass bereits 30 Stunden nach Impfung der Gastrieb beginnt, und dieser nach nur wenigen Tagen abgeschlossen ist. Dies bewirkt beim Boostern ein "Mitreissen" aller gasmäßig noch nicht aufgeschlossenen Bestandteile im Gärbrei.

Das wichtige dabei ist aber, dass der Massenanteil des Stoffgemisches, d.h. des Boosters, in Relation zur zu boosternden Gärbreimenge in einer bevorzugten Betriebsweise nur ca 0,1 bis 2,0 Gew% liegt.
Damit lässt sich zeigen, dass nach Abzug der im Booster eingeführten Energiemenge, die durch die erheblich geringen Mengen von Booster in Relation zum Gärbrei eingeschleust werden, der Gastrieb insbesondere im Nachfermentationsprozess signifikant erhöht ist. Dies liegt an der insbesondere auf die Nachfermentation adaptierte Mikrobiologie sowie der Anschubaktivität des Boosters.

In weiterer vorteilhafter Ausgestaltung ist daher vorgesehen, dass das Stoffgemisch aus aquatischen Pflanzen mit zugemischtem trockenem oder angetrocknetem Pflanzenmehl, sowie einem oder mehreren der nachfolgend genannten Stoffe vermischt ist, nämlich mit Fettsäuren oder Fettsäureestern, und/oder Eisenhydroxid, und/oder PH-Regulator, und/oder Zeolite, und/oder verseifte Rückstande aus der Lebensmittel- oder Speiseölproduktion, und/oder Enzyme, und/oder Mineralien, und/oder gemahlene Aktiv- und/oder Holzkohle, und/oder der in dem Verfahren nach Anspruch 7 genannnten bakteriologischen Impfmasse.
Das heisst, diese Ausgestaltung umfasst optional die Beimengung der bakteriologischen Impfmasse, die in dem genannten Brutreaktor erzeugt wird.

Alternativ dazu kann die besagte bakteriologische Impfmasse, die in dem genannten Brutreaktor erzeugt wird, auch direkt dem Haupt- oder Nachfermenter zugemischt werden, ohne dass er zuvor noch mit dem Booster vermischt wird.

In weiterer vorteilhafter Ausgestaltung ist angegeben, dass das trockene Pflanzenmehl Maismehl aus dem Fruchtstand der Maispflanze ist.

Alternativ dazu kann es aber ebenso vorteilhaft sein, dass das trockene Pflanzenmehl Mehl aus der gesamten Maispflanze gebildet ist.

Eine weitere Alternative ist, dass das Pflanzenmehl aus gemahlenem Getreide besteht.
In all diesen Fällen wird der resultierende Feuchtegehalt ohne eine thermomechanische Trocknung reduziert, so dass die aquatischen Pflanzen anschließend wieder in der Lage sind, in den auf diese Weise entwässerten Pflanzenkörper wieder besagte flüssigen Stoffe einzubringen.

Eine weitere vorteilhafte Ausgestaltung besteht darin, dass weiterhin Amylose und/oder Carbonsäure dem besagten Stoffgemisch, d.h. Biogasbooster zugesetzt wird. Diese unterstützen den Biogasprozess ebenfalls erheblich.

Ausgestaltungen der Erfindung sind in den Zeichnungen dargestellt und nachfolgend näher beschrieben.

Es zeigt:
Figur 1: Gesamtüberblick aller Verfahrensoptionen
Figur 2: separater Erbrütungsprozess von Bakterienstämmen

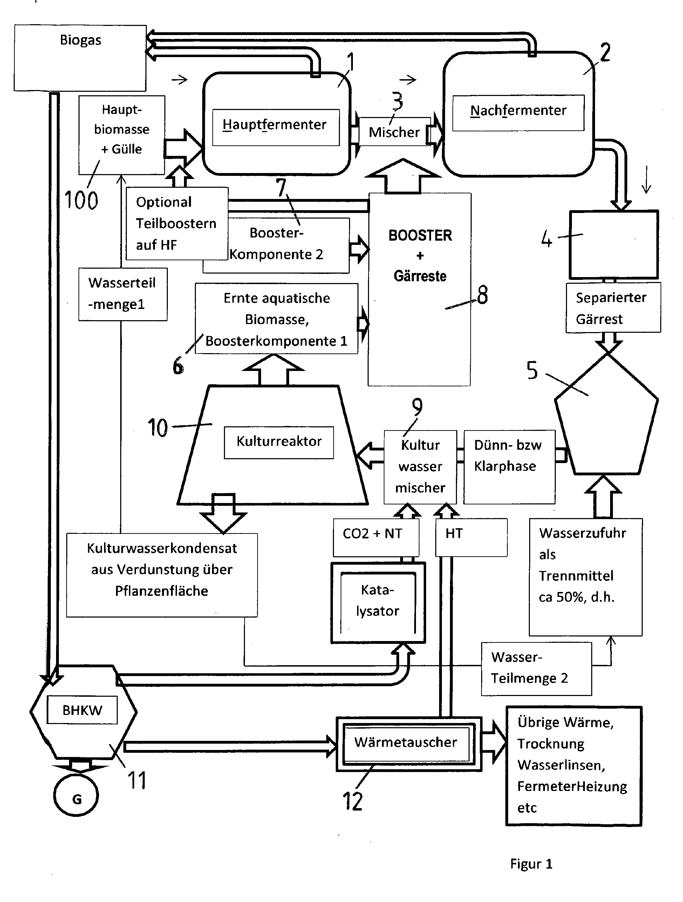

Figur 1 zeigt in einer Gesamtdarstellung alle möglichen Optionen beim Einsatz des bereits oben beschriebenen Stoffgemisches oder Boosters. Die hauptsächliche Biomasse 100 in Form von pflanzlichen oder auch anderen biogenen Biogassubstraten und/oder Güllen wird dem Hauptfermenter (Hf) 1 zugeführt und vergärt und Biogas freigesetzt. Der Methangehalt liegt üblicherweise bei 47% bis 52%. Nach Vergärung des Substrates im Hauptfermenter (Hf) 1 wird der teilausgegaste Gärbrei bekanntermaßen einem Nachfermenter (Nf) 2 zugeführt. Auch dort wird nochmals Biogas erzeugt. Erst dann werden die ausgegasten Gärreste aus dem Nachfermenter (Nf) 2 einem mechanischen Separator 4 zugeführt. Dieser trennt die festen Bestandteile von dem Gärbrei ab. Dabei wird eine erste Dick- oder Feststoffphase entnommen, die hier nicht mehr weiter dargestellt ist. Der separierte Gärbrei weist aber immer noch eine dünne breiartige Konsistenz auf und enthält außerdem noch gelöste Nährstoffe und Bestandteile.

Diese werden in einem nachfolgenden Fällungsprozess gefällt, und dabei ergibt sich eine Dünn- oder Klarphase, sowie eine hier auch nicht weiter dargestellte Flockenphase, die auch hier entnommen, d.h. mechanisch abgetrennt wird. Die Klarphase hingegen wird einem Kulturwassermischer 9 zur Herstellung von Kulturwasser für eine Kulturanlage 10 mit aquatischen Pflanzen zugeführt. Die Flockenphase wird ggfs mit der erstgenannten Dickphase vermischt und optional zur Herstellung von Kulturerde verwendet. Teilmengen davon können auch der Biogasanlage d.h. dem Hauptfermenter (Hf) 1 und/oder dem Nachfermenter (Nf) 2 zurückgeführt werden.

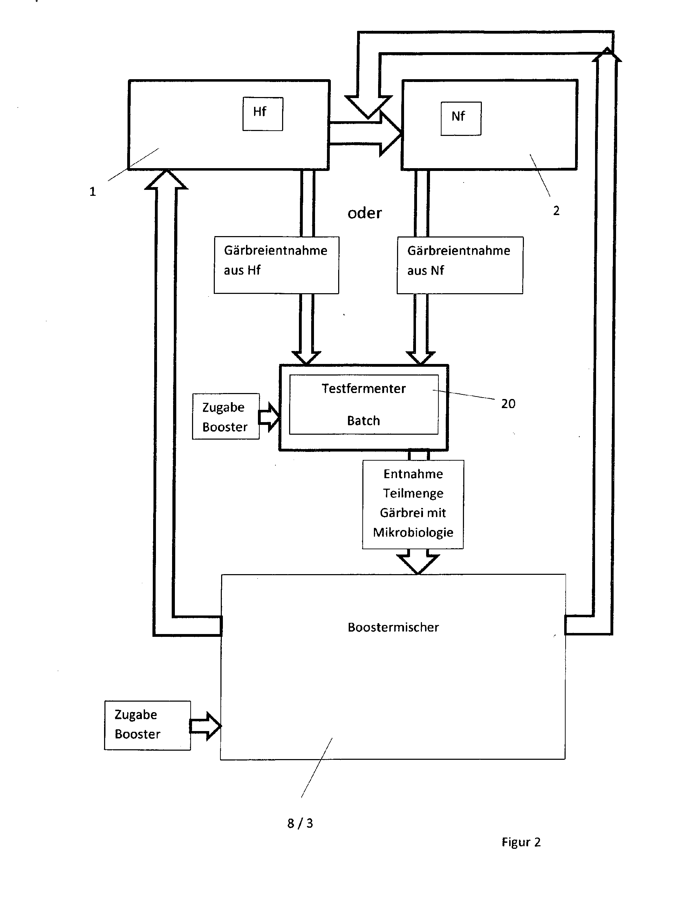

Im Kulturreaktor 10 werden nun aquatische Pflanzen, bspw Wasserlinsen in einem gestapelten Wannensystem kultiviert, vermehrt und regelmäßig abgeerntet. Diese Biomasse bildet sodann die erste Komponente 6 im Stoffgemisch/Biogasbooster. Als zweite Komponente 7 kommen nun die oben genannten Stoffe hinzu, und werden in dem Boostermischer 8 vermischt, ggfs mit Gärresten und/oder der oben genannten Dick- und/oder Flockenphase. Mit dieser wird auch eine mikrobiologische Zugabe beigefügt, die in dem in Figur 2 dargestellten, zeitlich parallelen Verfahren hergestellt wird.

Dies wird zusammen einem Zumischer 3 zugeführt. Das so erstellte Stoffgemisch/Booster 8 wird in den Überführungsstrom des Gärbreies vom Hauptfermenter (Hf) 1 in den Nachfermenter (Nf) 2 einfach mit injiziert, wodurch es zu einer gleichförmigen Vermischung des Boosters im Gärbrei kommt, ohne dass im Nachfermenter noch Rührwerke für eine Vermischung sorgen müssten.

Das besagte Stoffgemisch, d.h. der Biogasbooster sorgt nun im Nachfermenter für den oben bereits ausgeführte verstärkten Gastrieb in der so optimierten Nachvergärung.

Im Dauerbetrieb der Biogasanlage werden vorzugsweise täglich Boostermengen in den Überleitungsstrom des Gärbreis vom Hauptfermenter in den Nachfermenter mit injiziert. D.h. wenn die tägliche übergeleitete Gärbreimenge 30 to beträgt, so werden 0,5 bis 2 Gewichts% davon an Boostermenge mit injiziert. Nach etwa 30 bis 60 Tagen ist dann der Nachfermenter durchgeboostert. Danach wird täglich mit der übergeleiteten Gärbreimenge täglich nachgeboostert.
Es ist natürlich auch möglich, die gesamte Boostermenge auf einmal in den Nachfermenter zu bringen, und dann nur noch täglich wie beschrieben nachzuboostern.

Aus dem Kulturreaktor 10 fallen großen Mengen an Wasserkondensat an, die gesammelt und abgeführt werden. Diese können als Neutralwasser zur Einstellung des optimalen Feucht-/Trockensubstanzgehaltes im Gärbrei durch gesteuerte Zumischung herangezogen werden, und/oder zur Rückführung von Wasser als Trennungsmittel im Fällungsprozess der vorseparierten Gärreste.

Das Blockheizkraftwerk verarbeitet das Biogas zu Strom und Wärme. Die Wärme wird in einem geschlossenen Wärmekonzept dann wahlweise einer Trocknung der aquatischen Pflanzen, und zur Beheizung des Kulturreaktors verwertet. Durch die damit im Kulturreaktor einstellbaren tropischen Temperaturen wird eine erhöhte Populationsrate der aquatischen Pflanzen bewirkt. Durch den höheren Ertrag stehen höhere Biomassen zur Verfügung, und die Verdunstungsleistung des Kulturreaktors 10 wird ebenfalls erhöht.
Wie an diesem Schaubild zu sehen ist, entstehen somit keinerlei Reste mehr. Mit der Erhöhung der Verdunstungsleistung im besagten Wärmekonzept kann somit eine vollständige Entsorgung der Gärreste bewirkt werden.

Durch Recyclizierung von Wärme und Stoffen wird der Biogasprozess so erheblich im Gasertrag gesteigert, dass die eingangsseitig zugeführte Menge an Biomassensubstrat bei gleicher elektrischer Leistung der Anlage reduziert werden kann.

Damit können Biogasanlagen wieder wirtschaftlich und mit guten ökonomischen Erträgen betrieben werden.

Figur 2 zeigt das Verfahren, bei dem das besagte Stoffgemisch, d.h. der Biogasbooster auf eine jeweilige Biogasanlage appliziert hergestellt wird.
Dem Hauptfermenter (Hf) 1, oder dem Nachfermenter (Nf) 2 wird Gärbrei entnommen. Dieser wird einem separaten Testfermenter 20 zugeführt. Der Gärbrei wird zuvor noch einer chemischen und/oder biologischen Analyse unterzogen. Darauf wird das oben genannte Stoffgemisch oder Booster aus den oben genannten Komponenten zugeführt. Dabei erfolgt eine Vergärung in einem Batchverfahren. D.h. der Testfermenter 20 wird einmal mit Gärbrei und Biogasbooster gefüllt und hernach 2 bis 6 Wochen damit anaerob betrieben. Dadurch, dass es sich bei dem zugeführten Gärbrei um normalerweise ausgegasten oder teilausgegasten Gärbrei handelt, erfolgt durch die Zugaben des Boosters ein erneuter Nachvergärungsaufschluss des Gärbreis.
Dabei wird der Gastrieb und der Methangehalt gemessen, und durch geringfügige Zugaben von einer oder mehreren im Stoffgemisch oder Biogasbooster genannten Komponenten nochmals ergänzt und mit in den Testfermenter eingespeist, so, dass Gastrieb und Methangehalt maximal gehalten werden.
Die dabei zugeführten Komponenten, werden bei der finalen Zusammensetzung des zu diesem Gärbrei optimierten Biogasboosters mengemäßig mit berücksichtigt.

Der Testfermenter 20 ist keineswegs eine übliche Laboranlage mit 2 bis 10 liter Fassungvermögen, sondern eine Anlage mit ca 1000 ltr Volumen. Damit und nur damit ist eine hinreichende Hochskalierung auf Biogasanlagen mit 2.000 m3 möglich. Laboranlagen aus Glaszylindern mit 2 bis 10 liter Volumen eignen sich dafür nicht.

Am Ende des Fermentationsprozesses im Testfermenter 20 werden dann Teilmengen dieser im Testfermenter durch die gezielte erfindungsgemäße Boosterung populierten Bakterienstämme als Impfmasse mit entnommen, und dem Biogasbooster beigemengt, oder bei der Zuführung in den Prozess der Biogasanlage mit zugemischt.

Diese im Testfermenter unter Boosterbedingungen erbrüteten Bakterienstämme sind auf diese Weise genau auf die zu boosternde Biogasanlage individuell appliziert. Mit dieser erfindungsgemäßen Vorgehensweise wird bewirkt, dass die Populationsverhältnisse verschiedener Bakterienstämme in der Mischpopulation durch die Testfermentation mit Boosterapplikation so verändert werden, dass hernach die für den finalen Gasaufschluss notwendigen Bakterien im Verhältnis überpopuliert sind. Werden diese nun bspw dem Nachfermenter (Nf) 2 zusammen mit dem Biogasbooster zugeführt, so steigt damit der Gastrieb und der Methangehalt im Nachfermenter deutlich. Das heisst mit anderen Worten, dass der Nachfermenter so optimiert wird, dass die anfallende Biogasmenge gesteigert wird. In Folge kann damit die elektrische Anschlussleistung im BHKW erhöht werden, oder man kann die Menge an Hauptgärsubstrat am Eingang des Hauptfermenters reduzieren bei gleicher abgegebener elektrischer Leistung.
Mit anderen Worten erhöht das erfindungsgemäße Verfahren die nutzbare Gasausbeute, weil bei bekannten Biogasanlagen im angeblich ausgegasten Gärbrei noch immer nicht vergaste Bestandteile enthalten werden, und auf Deponien ungenutzt bleiben.
Hinzu kommt, dass eine Deponie für Gärreste bei diesem Verfahren ebenfalls entfällt.
Neben dem höheren Gasertrag, bzw geringerem Substratbedarf entfallen somit Deponie- und Entsorgungskosten. Insgesamt entsteht so ein weiteres Ertragsplus an der Biogasanlage.

Wichtig ist auch, dass den geernteten aquatischen Pflanzen ohne Antrockung sofort trockenes Pflanzenmehl zugemischt wird, die der Frischmasse der aquatischen Pflanzen sofort Wasser entziehen, ohne dass man eine Antrocknung der aquatischen Pflanzen vornehmen muss. Der angetrocknete Pflanzenkörper der aquatischen Pflanzen ist somit in der erfindungsgemäßen Weise aufnahmefähig für flüssige Stoffe, wie oben genannt beizufügen die vom angetrockneten Pflanzenkörper der aquatischen Pflanzen dann im Maß dieser Antrocknung wieder aufgenommen werden.

### Bezugszeichen

- 1: Hauptfermenter
- 2: Nachfermenter
- 3: Zumischer
- 4: Separator
- 5: Fällung
- 6: erste Boosterkomponente
- 7: zweite Boosterkomponente
- 8: Boostermischer
- 9: Kulturwassermischer
- 10: Kulturreaktor
- 11: BHKW
- 12: Wärmetauscher

- 20: Testfermenter
- 100: Hauptbiomasse/Biogassubstrat/Gülle

## Patentansprüche

1. Verfahren zum Betrieb einer Biogasanlage bei welcher landwirtschaftliche Gülle und/oder in einem vorherigen Gärprozess bereits teilausgegaster Gärbrei, und/oder Klärschlamm, und/oder Abfälle aus der Lebensmittelproduktion als Gärssubstrat in mindestens einem Gärprozess vergärt oder nachvergärt wird, wobei der landwirtschaftlichen Gülle und/oder dem Gärbrei oder dem teilausgegasten Gärbrei, und/oder dem Klärschlamm, und/oder den Abfällen aus der Lebensmittelproduktion ein die Biogasproduktion unterstützendes Stoffgemisch, d.h. Biogasbooster beigefügt wird, welches zuvor hergestellt wird, und das/der Stoffgemisch/Biogasbooster aquatischen Pflanzen mit Beimengungen von Fettsäuren und/oder Fettsäureestern enthält, sowie ein Verfahren zur Herstellung eines solchen Boosters, **dadurch gekennzeichnet,**
**dass** zusätzlich Eisenhydroxid, und/oder PH-Regulator, und/oder Zeolite, und/oder verseifte Rückstande aus der Lebensmittel- oder Speiseölproduktion, und/oder Enzyme, und/oder Mineralien, und/oder gemahlene Aktiv- und/oder Holzkohle dem besagten Stoffgemisch hinzugefügt wird.

2. Verfahren nach Anspruch 1,
**dadurch gekennzeichnet,**
**dass** dieses, die Biogasproduktion unterstützende Stoffgemisch, einem Nachfermentationsprozess einer Biogasanlage zugeführt wird.

3. Verfahren nach Anspruch 1,
**dadurch gekennzeichnet,**
**dass** dieses, die Biogasproduktion unterstützende Stoffgemisch, einem Hauptfermentationsprozess einer Biogasanlage zugeführt wird.

4. Verfahren nach Anspruch 2 oder 3,
**dadurch gekennzeichnet,**
**dass** das die Biogasproduktion unterstützende Stoffgemisch dem Haupt- oder Nachfermenter der Biogasanlage in einem Zeittakt von 6 bis 30 Stunden in wiederholten Dosen zugeführt wird, derart dass der Methangastrieb aus dem Gärbrei dauerhaft angehoben und gehalten wird, und eine Biomassezufütterung zeitlich parallel kontinuierlich so nachgeführt wird, dass der gleiche Methangastrieb bei mengenmäßig reduzierter Biomassenzufütterung erhalten bleibt.

5. Verfahren nach einem der Ansprüche 1 bis 4,
**dadurch gekennzeichnet,**
**dass** bei der Produktion der aquatischen Pflanzen die beim Betrieb der Biogasanlage und/oder die beim Betrieb eines BHKWs anfallende Abwärme zur Beheizung einer Kulturanlage zur Produktion der aquatischen Pflanzen eingesetzt wird, und dass das in der Biogasproduktion oder beim BHKW-Betrieb anfallende CO2 der Kulturanlage als vorzugeweise gasförmiger Düngestoff zugeführt.

6. Verfahren zur Herstellung eines die Biogasproduktion in einer Biogasanlage unterstützenden Stoffgemisches oder Boosters, welches im wesentlichen aus einem Gemisch besteht, dessen erste Komponente aus einer oder mehreren aquatischen aquatischen Pflanzen besteht, und dessen zweite Komponente Fette, und/oder Fettsäuren besteht,
**dadurch gekennzeichnet,**
**dass** die aquatischen Pflanzen zunächst als Frischmasse eingesetzt wird, die durch Zugabe eines trockenen oder teilgetrockneten hydrophilen Pflanzenmehles teilentwässert werden, und zu einem Gemisch mit entsprechend resultierendem Trockensubstanzgehalt vermengt wird, dessen resultierender Trockensubstanzgehalt höher ist als der Trockensubstanzgehalt der eingesetzten aquatischen Pflanzen.

7. Verfahren nach Anspruch 6,
**dadurch gekennzeichnet,**
**dass** zur Herstellung des Stoffgemisches oder Boosters folgende Stoffe hernach mit zugemischt werden,
Eisenhydroxid, und/oder PH-Regulator, und/oder Zeolite, und/oder verseifte Rückstande aus der Lebensmittel- oder Speiseölproduktion, und/oder Enzyme, und/oder Mineralien, und/oder gemahlene Aktiv- und/oder Holzkohle.

8. Verfahren zum Betrieb einer Biogasanlage bei welcher landwirtschaftliche Gülle und/oder in einem vorherigen Gärprozess bereits teilausgegaster Gärbrei, und/oder Klärschlamm, und/oder Abfälle aus der Lebensmittelproduktion als Gärssubstrat in mindestens einem Gärprozess vergärt oder nachvergärt wird, wobei der landwirtschaftlichen Gülle und/oder dem Gärbrei oder dem teilausgegasten Gärbrei, und/oder dem Klärschlamm, und/oder den Abfällen aus der Lebensmittelproduktion ein die Biogasproduktion unterstützendes Stoffgemisch beigefügt wird, welches zuvor hergestellt wird, **dadurch gekennzeichnet, dass** der Biogasanlage zunächst ausgegaster oder teilausgegaster Gärbrei entnommen wird, und in einem gesonderten bakteriologischen Brutgefäß eingebracht und das dem besagte Stoffgemisch hinzugeführt wird, dass in einem Batchverfahren zunächst der Gärbrei zusammen mit dem besagten Stoffgemisch über einen Zeitraum von 1 bis 45 Tagen bei einer Temperatur von etwa 30°C bis etwa 42 °C oder höher anaerob kultiviert wird, derart, dass sich diejenigen Bakterienstämme aus dem Gemisch vorhandender Bakterienstämme überproportional entwickeln, die genau an das Gemisch des entnommenen Gärbreies mit dem Stoffgemisch adaptiert haben, und dass diesem so bakteriell erbrüteten Gärbrei Teilmengen entnommen werden und als bakteriologische Impfmasse in das finale Stoffgemisch oder direkt in den Haupt- oder Nachfermentationsprozess mit eingemischt wird.

9. Stoffgemisch zur Erhöhung der Biogasproduktion in Biogasanlagen,
**dadurch gekennzeichnet,**
**dass** das Stoffgemisch aus aquatischen Pflanzen mit zugemischtem trockenem.oder angetrocknetem Pflanzenmehl, sowie einem oder mehreren der nachfolgend genannten Stoffe vermischt ist, nämlich mit Fettsäuren oder Fettsäureestern, und/oder Eisenhydroxid, und/oder PH-Regulator, und/oder Zeolite, und/oder verseifte Rückstande aus der Lebensmittel- oder Speiseölproduktion, und/oder Enzyme, und/oder Mineralien, und/oder gemahlene Aktiv- und/oder Holzkohle, und/oder der in dem Verfahren nach Anspruch 8 genannnten bakteriologischen Impfmasse.

10. Stoffgemisch nach Anspruch 9,
**dadurch gekennzeichnet,**
**dass** das trockene Pflanzenmehl Maismehl aus dem Fruchtstand der Maispflanze ist.

11. Stoff oder Stoffgemisch, nach Anspruch 9,
**dadurch gekennzeichnet,**
**dass** das trockene Pflanzenmehl Mehl aus der gesamten Maispflanze ist.

12. Stoff oder Stoffgemisch, nach Anspruch 9,
**dadurch gekennzeichnet,**
**dass** das Pflanzenmehl aus gemahlenem Getreide besteht.

13. Stoff oder Stoffgemisch, nach einem der Ansprüche 9 bis 12,
**dadurch gekennzeichnet,**
**dass** weiterhin Amylose und/oder Carbonsäure dem besagten Stoffgemisch, d.h. Biogasbooster zugesetzt wird.
